# EUROPEAN PATENT APPLICATION

(11) **EP 2 105 146 A1**
(43) Date of publication of application: **30.09.2009**
(21) Application number: 08168538.0
(22) Date of filing: 06.11.2008
(51) Int. Cl.: A61K 47/48, C12Q 1/68, C07D 211/62, C07C 245/08

(54) **Nucleic-acid triggered release of molecules**

(30) Priority: 07.11.2007 JP 2007289694
(71) Applicant: Riken, Wakou-shi, Saitama 351-0198 (JP)
(72) Inventor: ABE, Hiroshi, Saitama Saitama 351-0198 (JP); ITO, Yoshihiro, Saitama Saitama 351-0198 (JP); SHIBATA, Aya, Saitama Saitama 351-0198 (JP); KODAMA, Hiroaki, Saitama Saitama 351-0198 (JP)
(74) Representative: Polypatent

(57) **Abstract**

It is an object of the present invention to provide a method for highly selectively releasing a molecule of interest such as an agent at a desired site while suppressing the influence of catabolic enzymes existing *in vivo*. The present invention provides a method for releasing a molecule of interest, which comprises steps of: hybridizing each of an "electron donor-first nucleic acid probe" molecule formed by binding an electron donor structure to a first nucleic acid probe having a nucleotide sequence complementary to a portion of a target nucleic acid sequence and a "molecule of interest-electron acceptor-second nucleic acid probe" molecule formed by binding an electron acceptor structure having a molecule of interest and an azide group to a second nucleic acid probe that has a nucleotide sequence complementary to said target nucleic acid sequence and differing from that of said first nucleic acid probe, to said target nucleic acid sequence; and allowing said "electron donor-first nucleic acid probe" molecule to act on said "molecule of interest-electron acceptor-second nucleic acid probe" molecule, so as to release said molecule of interest.

SEQUENCE LISTING
<110>  RIKEN
<120>  Method for releasing an objective molecule based on a target
       nucleic acid sequence
<130>  FA8257A
<160>  4
<170>  PatentIn version 3.4
<210>  1
<211>  27
<212>  DNA
<213>  Artificial Sequence
<220>
<223> Description of Artificial Sequence: Synthetic DNA
<400>  1
taagcagagt tcaaaagcac ttcagcg                                          27
<210>  2
<211>  15
<212>  DNA
<213>  Artificial Sequence
<220>
<223> Description of Artificial Sequence: Synthetic DNA
<400>  2
gctggcggtc tgggt                                                       15
<210>  3
<211>  15
<212>  DNA
<213>  Artificial Sequence
<220>
<223> Description of Artificial Sequence: Synthetic DNA
<400>  3
gtttccctct tcacg                                                       15
<210>  4
<211>  15
<212>  DNA
<213>  Artificial Sequence
<220>

<223> Description of Artificial Sequence: Synthetic DNA
<400> 4
gccttctccc gaagt 15

SEQUENCE LISTING

<110>  RIKEN

<120>  Method for releasing an objective molecule base on a target

       nucleic acid sequence

<130>  P22275EP

<150>  JP2007-289694
<151>  2007-11-07

<160>  7

<170>  PatentIn version 3.3

<210>  1
<211>  27
<212>  DNA
<213>  Artificial

<220>
<223>  primer

<400>  1
taagcagagt tcaaaagcac ttcagcg
   27

<210>  2
<211>  15
<212>  DNA
<213>  Artificial

<220>
<223>  primer

<400>  2
gctggcggtc tgggt
   15

<210>  3
<211>  15
<212>  DNA
<213>  Artificial

<220>
<223>  primer

<400>  3

gtttccctct tcacg
    15


<210>  4
<211>  15
<212>  DNA
<213>  Artificial

<220>
<223>  primer

<400>  4
gccttctccc gaagt
    15


<210>  5
<211>  8
<212>  DNA
<213>  Artificial

<220>
<223>  primer

<400>  5
aagtgctt
     8


<210>  6
<211>  8
<212>  DNA
<213>  Artificial

<220>
<223>  primer

<400>  6
ttgaactc
     8


<210>  7
<211>  31
<212>  DNA
<213>  Artificial

<220>
<223>  primer

<400>  7

cgtgaagagg gaaacaaccc agaccgccag c
31

## Description

### TECHNICAL FIELD

The present invention relates to a method for selectively releasing a molecule of interest, which comprises releasing a molecule of interest such as an agent, depending on a target nucleic acid sequence. More specifically, the present invention relates to a method for releasing a molecule of interest in the neighborhood of a target nucleic acid sequence, which is characterized in that it comprises: hybridizing a first nucleic acid probe wherein an electron donor structure has bound to a portion of the target nucleic acid sequence with a second nucleic acid probe to which an electron acceptor structure having a molecule of interest and an azide group have bound in the neighborhood of the first nucleic acid probe; and transferring electrons from the electron donor to the electron acceptor, so as to release the molecule of interest. Furthermore, the present invention relates to a method for detecting a target nucleic acid sequence utilizing an FRET (fluorescence resonance energy transfer) effect, which comprises hybridizing the first nucleic acid probe and the second nucleic probe with the target nucleic acid sequence, as stated above, using a molecule of interest as a quencher and also using the aforementioned second nucleic acid probe having an electron acceptor structure, to which a fluorescent agent has also bind. Still further, the present invention relates to a novel electron donor and a novel electron acceptor used in the aforementioned method for releasing a molecule of interest and in the aforementioned method for detecting a target nucleic acid sequence, and a chemical structure wherein the electron donor and the electron acceptor are bound to the aforementioned nucleic acid probes, respectively.

### BACKGROUND ART

As a method for releasing an agent to a specific site in a living body, there has been widely used a method of including an agent with a carrier such as a polymer micelle or an inorganic compound so as to control the sustained release property and absorption property of the agent in the living body, namely, what is called a drug delivery system. However, when such a carrier is used, the action site of an agent is recognized depending only on the size of the carrier. Thus, such a drug delivery system has been problematic in that the selectivity of recognition of the action site is not high.

As a method for solving the aforementioned problem, Taylor et al. have reported a method of releasing an agent by recognizing a nucleic acid sequence in a cell (please see US Patent Application Laid-Open No. 2003/0060441 and Taylor, J. et al. (2000) Proc. Natl. Acad. Sci., 97, 11159-11163, for example). However, in the case of the method of Taylor et al., since a chemical mechanism using the hydrolysis of ester easily affected by various enzymes existing *in vivo* has been adopted as an agent-releasing mechanism, sufficient selectivity could not be obtained.

On the other hand, as a method for detecting a specific target nucleic acid sequence, there has been widely used a hybridization method using a nucleic acid probe, which has a nucleotide sequence complementary to the target nucleic acid sequence and is labeled with a fluorescent substance such as fluorescein, tetramethylrhodamine, Cy3, or Cy5. However, a fluorescent nucleic acid probe labeled with such a fluorescent substance has a high background fluorescent signal, and thus it has been difficult to conduct a highly sensitive measurement.

### DISCLOSURE OF THE INVENTION

It is an object of the present invention to solve the aforementioned problems of the prior art techniques. In other words, it is an object of the present invention to provide a method for highly selectively releasing a molecule of interest such as an agent at a desired site, and particularly in a cell of an *in vivo* or *in vitro* system that constitutes the desired site, while suppressing the influence of catabolic enzymes existing *in vivo*. In addition, it is another object of the present invention to provide a method for detecting a target nucleic acid sequence by utilizing an FRET effect, which is a stable, highly selective and highly sensitive method enabling detection of a trace amount of the target nucleic acid sequence. Moreover, it is a further object of the present invention to provide molecules having both a novel electron donor and a nucleic acid probe, and molecules having both a novel electron acceptor and a nucleic acid probe, which are able to provide the aforementioned method for releasing a molecule of interest and the aforementioned method for detecting a target nucleic acid sequence.

As a result of intensive studies directed towards achieving the aforementioned objects, the present inventors have succeeded in synthesizing an electron acceptor structure having an azide group, which is not decomposed by catabolic enzymes existing *in vivo* and that does not nonselectively release a bound molecule of interest. Moreover, the inventors have created: molecules formed by binding a first nucleic acid probe to an electron donor structure, which are capable of hybridizing with a portion of a target nucleic acid sequence; and molecules formed by binding a second nucleic acid probe, an electron acceptor molecule having an azide group, and the aforementioned molecule of interest, which are capable of hybridizing with the first nucleic acid probe at a certain distance. The inventors have then hybridized each of these molecules with the target nucleic acid sequence, and as a result, they have discovered that the molecule of interest and azidomethyl can be released from the aforementioned electron acceptor structure. Furthermore, they have also discovered the following: When a quencher is used as such a molecule of interest, and a fluorescent agent such as fluorescein is allowed to bind to the nucleic acid probe binding to the electron acceptor structure, this structure does not emit fluorescence on its own. However, when the aforementioned nucleic acid probe binding to the electron acceptor structure, together with a nucleic acid probe binding to an electron donor structure, hybridizes with a target nucleic acid sequence, the quencher is released and thereby the fluorescent agent binding to the nucleic acid probe emits fluorescence. By detecting this fluorescence, the target nucleic acid sequence can be detected. Based on these findings, the present inventors have completed the present invention.

Specifically, the present invention provides a method for releasing a molecule of interest, which comprises steps of:
hybridizing each of an "electron donor-first nucleic acid probe" molecule formed by binding an electron donor structure to a first nucleic acid probe having a nucleotide sequence complementary to a portion of a target nucleic acid sequence and a "molecule of interest-electron acceptor-second nucleic acid probe" molecule formed by binding an electron acceptor structure having a molecule of interest and an azide group to a second nucleic acid probe that has a nucleotide sequence complementary to a nucleic acid sequence in the neighborhood separated at a certain distance from a portion of said target nucleic acid sequence and differing from that of said first nucleic acid probe, to said target nucleic acid sequence; and
allowing said electron donor structure to act on said "molecule of interest-electron acceptor" structure, so as to release said molecule of interest.

Preferably, in the chemical structure of the present invention for releasing a molecule of interest,
a nucleotide sequence complementary to the nucleotide sequence of said second nucleic acid probe is located closer to the 3'-terminal side of said target nucleic acid sequence than a nucleotide sequence complementary to the nucleotide sequence of said first nucleic acid probe is;
said electron donor structure binds to the 5'-terminal portion of said first nucleic acid probe in said electron donor-first nucleic acid probe molecule; and
said electron acceptor structure binds to the 3'-terminal portion of said second nucleic acid probe in said molecule of interest-electron acceptor-second nucleic acid probe molecule.

Preferably, in the chemical structure of the present invention for releasing a molecule of interest,
a nucleotide sequence complementary to the nucleotide sequence of said second nucleic acid probe is located closer to the 5'-terminal side of said target nucleic acid sequence than a nucleotide sequence complementary to the nucleotide sequence of said first nucleic acid probe is;
said electron donor structure binds to the 3'-terminal portion of said first nucleic acid probe in said electron donor-first nucleic acid probe molecules; and
said electron acceptor structure binds to the 5'-terminal portion of said second nucleic acid probe in said molecule of interest-electron acceptor-second nucleic acid probe molecules.

Preferably, in the chemical structure of the present invention for releasing a molecule of interest, a nucleotide sequence complementary to the nucleotide sequence of said second nucleic acid probe is located directly next to or 1 to 20 nucleotides away from a nucleotide sequence complementary to the nucleotide sequence of said first nucleic acid probe.

In the chemical structure of the present invention for releasing a molecule of interest, said electron donor structure is preferably a structure comprising a reducing agent, and is more preferably a structure comprising a diphenylphosphine group.

Further preferably, in the chemical structure of the present invention for releasing a molecule of interest, said electron acceptor structure is represented by the following formula (1): 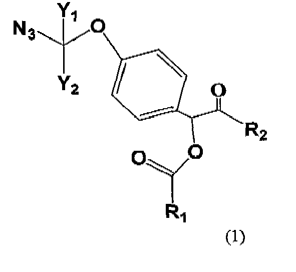 (wherein, in the above formula (1), each of Y₁ and Y₂ independently represents a hydrogen atom, an alkyl group containing 1 to 6 carbon atoms, an alkoxy group containing 1 to 6 carbon atoms, an aryl group containing 6 to 10 carbon atoms, or a cyano group; R₁ represents a residue of the molecule of interest; and R₂ represents a reactive group for binding to a nucleic acid.)

Preferably, in the chemical structure of the present invention for releasing a molecule of interest, the above R₂ is a reactive group represented by the following formula (2), but is not limited thereto.

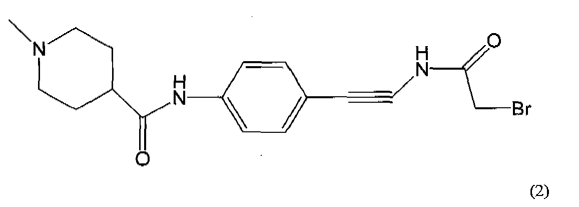

In the method of the present invention for releasing a molecule of interest, said target nucleic acid is DNA or RNA.

In the method of the present invention for releasing a molecule of interest, the molecule of interest is a poison, a medical pharmaceutical, or a reagent used for various purposes. An example of such a reagent is a quencher. More preferred examples include IPTG (isopropyl β-D-1-thiogalactopyranoside) and dabcyl.

In another aspect, the present invention provides a method for detecting a target nucleic acid, which comprises:
a step of hybridizing each of an "electron donor-first nucleic acid probe" molecule formed by binding an electron donor structure to a first nucleic acid probe having a nucleotide sequence complementary to a portion of a target nucleic acid sequence, and a "quencher-electron acceptor-fluorescent-agent-bound second nucleic acid probe" molecule (hereinafter referred to as "quencher-electron acceptor-fluorescent agent probe" at time) formed by binding an electron acceptor structure having a quencher and an azide group to a second nucleic acid probe that has a nucleotide sequence complementary to a nucleic acid sequence in the neighborhood separated at a certain distance from a portion of said target nucleic acid sequence and differing from that of said first nucleic acid probe and a fluorescent agent, to said target nucleic acid sequence, and then allowing said electron donor structure to act on said quencher-electron acceptor-fluorescent agent structure, so as to release said quencher; and
a step of measuring the fluorescence of a complex obtained by said hybridization.

Preferably, in the chemical structure of the present invention for detecting a target nucleic acid sequence,
a nucleotide sequence complementary to the nucleotide sequence of said second nucleic acid probe is located closer to the 3'-terminal side of said target nucleic acid sequence than a nucleotide sequence complementary to the nucleotide sequence of said first nucleic acid probe is;
said electron donor structure binds to the 5'-terminal portion of said first nucleic acid probe in said electron donor-first nucleic acid probe molecules; and
said electron acceptor structure binds to the 3'-terminal portion of said second nucleic acid probe in said quencher-electron acceptor-fluorescent agent probe.

Preferably, in the chemical structure of the present invention for detecting a target nucleic acid sequence,
a nucleotide sequence complementary to the nucleotide sequence of said second nucleic acid probe is located closer to the 5'-terminal side of said target nucleic acid sequence than a nucleotide sequence complementary to the nucleotide sequence of said first nucleic acid probe is;
said electron donor structure binds to the 3'-terminal portion of said first nucleic acid probe in said electron donor-first nucleic acid probe molecules; and
said electron acceptor structure binds to the 5'-terminal portion of said second nucleic acid probe in said quencher-electron acceptor-fluorescent agent probe.

Preferably, in the chemical structure of the present invention for detecting a target nucleic acid sequence, a nucleotide sequence complementary to the nucleotide sequence of said second nucleic acid probe is located directly next to or 1 to 20 nucleotides away from a nucleotide sequence complementary to the nucleotide sequence of said first nucleic acid probe.

In the chemical structure of the present invention for detecting a target nucleic acid sequence, said electron donor structure is preferably a structure comprising a reducing agent, and is more preferably a structure comprising a diphenylphosphine group.

Preferably, in the method of the present invention for detecting a target nucleic acid sequence, said electron acceptor structure is a compound represented by the following formula (3): 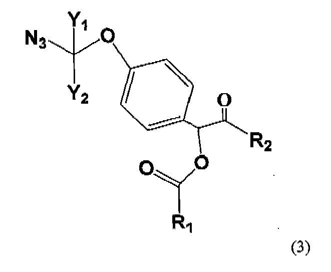 (wherein, in the above formula (3), each of Y₁ and Y₂ independently represents a hydrogen atom, an alkyl group containing 1 to 6 carbon atoms, an alkoxy group containing 1 to 6 carbon atoms, an aryl group containing 6 to 10 carbon atoms, or a cyano group; R₁ represents a residue of the quencher; and R₂ represents a reactive group for binding to a nucleic acid.)

Preferably, in the method of the present invention for detecting a target nucleic acid sequence, the above R₂ is a reactive group represented by the following formula (4):

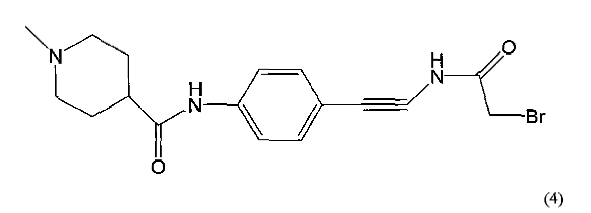

In the method of the present invention for detecting a target nucleic acid sequence, said target nucleic acid is preferably DNA or RNA.

In the method of the present invention for detecting a target nucleic acid, the quencher is preferably dabcyl.

In the method of the present invention for detecting a target nucleic acid, the fluorescent agent is preferably fluorescein.

In a further aspect, the present invention provides a compound represented by the following formula (5): 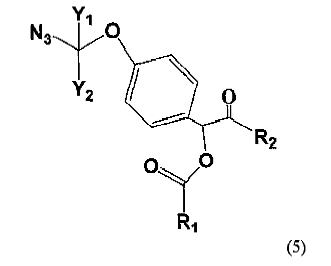 (wherein, in the above formula (5), each of Y₁ and Y₂ independently represents a hydrogen atom, an alkyl group containing 1 to 6 carbon atoms, an alkoxy group containing 1 to 6 carbon atoms, an aryl group containing 6 to 10 carbon atoms, or a cyano group; R₁ represents a residue of the molecule of interest; and R₂ represents a hydrogen atom, a halogen atom, or a reactive group for binding to a nucleic acid.)

Preferably, in the compound represented by the above formula (5) of the present invention, the above R₁ is a quencher, and is more preferably represented by the following formula (6):

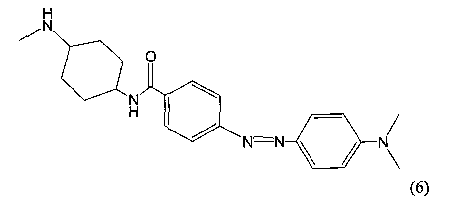

In a further aspect, the present invention provides a compound represented by the following formula (7):

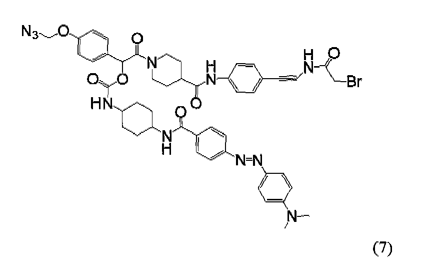

In a further aspect, there is provided the compound of the present invention to be used in the method of the present invention for releasing a molecule of interest or in the method of the present invention for detecting a target nucleic acid sequence.

The present invention provides a method for releasing a molecule of interest, which comprises detecting a target nucleic acid sequence using "electron donor-first nucleic acid probe" molecule and "molecule of interest-electron acceptor-second nucleic acid probe" molecule. The method of the present invention for releasing a molecule of interest is able to highly selectively release a molecule of interest to a specific site based on genetic information without being affected by catabolic enzymes existing *in vivo*. Accordingly, if the method of the present invention for releasing a molecule of interest is applied, a therapeutic agent for treating various diseases can be produced using nucleic acid probes targeting for various disease genes expressing in cells, and also using, as a molecule of interest, a poison for locally destroying abnormal cells, an agent for normalizing such abnormal cells, etc. That is to say, the present invention relates to an extremely useful technique that becomes a base for production of a therapeutic agent that directly acts on cells abnormalized by a certain disease and has few side effects. Furthermore, the present invention provides a method for detecting a target nucleic acid sequence, wherein a quencher is used as a molecule of interest and a fluorescent agent is allowed to bind to a nucleic acid probe, to which an electron acceptor structure having a quencher and an azide group have bound. Since the method of the present invention for detecting a target nucleic acid sequence is not affected by decomposition *in vivo* and thus it has a high signal/background ratio, it enables highly sensitive gene detection. At the same time, this method also enables gene detection imaging in a cell and in a living body. Further, in the method of the present invention for detecting a target nucleic acid sequence, a specific disease gene is used as such a target nucleic acid sequence. Thus, it can be expected as a diagnostic agent for diagnosing a specific disease without error. Still further, since it is not necessary to use other reagents or enzymes in the present invention, it is simple and inexpensive. It becomes possible to detect a gene not only in a test tube, not also in a cell or in a living body. Still further, the method of the present invention for releasing a target gene is highly safe (active for a long period of time) and highly sensitive. The present method enables amplification of a trace amount of gene signal and the observation thereof. These effects of the present invention can be actually obtained using the compound of the present invention.

### BRIEF DESCRIPTION OF THE DRAWINGS

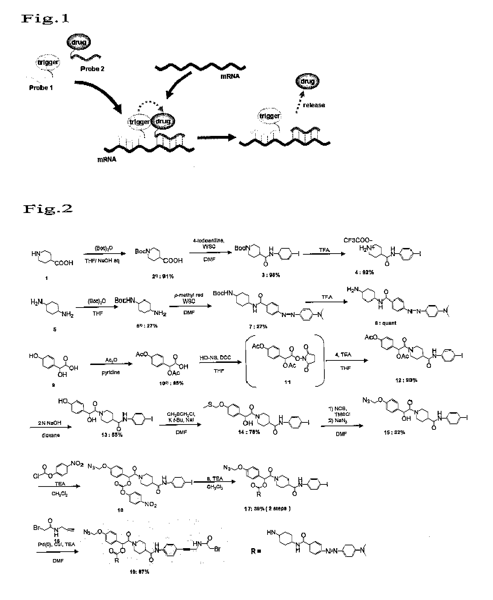

Figure 1 shows the summary of the method of the present invention for releasing a molecule of interest, which comprises hybridizing each of a first nucleic acid probe ("probe 1" in the figure) having an electron donor structure ("trigger" in the figure) and a second nucleic acid probe ("probe 2" in the figure) having an electron acceptor structure having a molecule of interest ("drug" in the figure) and an azide group, to a target nucleic acid sequence ("mRNA" in the figure), and allowing probe 1 to act on probe 2 to cause a change in the electron acceptor structure, thereby releasing the molecule of interest.
Figure 2 shows the organic synthesis scheme of compound 19.

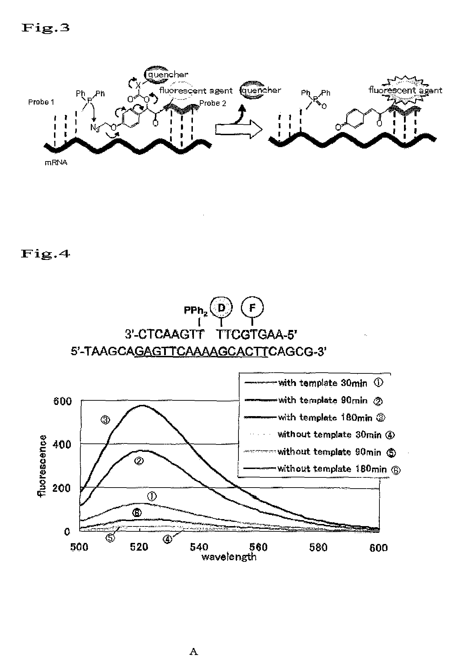

Figure 3 shows the summary of the method of the present invention for detecting a target nucleic acid sequence, which comprises hybridizing each of a first nucleic acid probe ("probe 1" in the figure) having an electron donor structure and a second nucleic acid probe ("probe 2" in the figure), to which a quencher has bound and which has an electron acceptor structure having an azide group ("drug" in the figure) and a fluorescent agent, to a target nucleic acid sequence ("mRNA" in the figure), and allowing probe 1 to act on probe 2 to cause a change in the electron acceptor structure, thereby releasing a molecule of interest.
Figure 4A shows a DNA probe formed by binding compound 19 having dabcyl and an azide group ("D" in the figure) to a fluorescent agent ("F" in the figure), a DNA probe formed by binding a triphenylphosphine group ("PPH₂" in the figure) to the 5'-terminal side, and a DNA template as shown in SEQ ID NO: 1 of the sequence listing. Figure 4B shows the results of a fluorometric measurement obtained after the reaction of the aforementioned DNA probe set with the DNA template.
Figure 5 shows the release of an agent in *E. coli* based on genetic information.
Figure 6 shows the results of an FAC measurement.

### BEST MODE FOR CARRYING OUT THE INVENTION

The embodiments of the present invention will be described in detail below.

The method of the present invention for releasing a molecule of interest is a method for releasing a molecule of interest, which comprises steps of:
hybridizing each of an "electron donor-first nucleic acid probe" molecule formed by binding an electron donor structure to a first nucleic acid probe having a nucleotide sequence complementary to a portion of a target nucleic acid sequence and a "molecule of interest-electron acceptor-second nucleic acid probe" molecule formed by binding an electron acceptor structure having a molecule of interest and an azide group to a second nucleic acid probe that has a nucleotide sequence complementary to a nucleic acid sequence in the neighborhood separated at a certain distance from a portion of said target nucleic acid sequence and differing from that of said first nucleic acid probe, to a portion of said target nucleic acid sequence and a nucleic acid sequence in the neighborhood separated at a certain distance from the portion of said target nucleic acid sequence, respectively; and allowing said electron donor structure to act on said molecule of interest-electron acceptor structure, so as to release said molecule of interest. The summary of the method of the present invention for releasing a molecule of interest is shown in Figure 1.

The term "molecule of interest" is used in the present specification to mean a molecule intended to be released to a specific site in a living body. The type of such a molecule of interest is not particularly limited. Preferred examples of such a molecule of interest include a medical pharmaceutical agent, a poison, an antibody, a vaccine, and various types of reagents including a quencher. Preferred examples of a pharmaceutical agent include an anticancer agent, an antiallergic agent, an anti-infective agent, an antirheumatic agent, an anti-neurogenic disease agent, an anti-blood disease agent, an anti-metabolic agent, and an anti-bone marrow disease agent. A preferred example of a poison is a poison that acts on a nucleic acid, a protein or a cell to locally destroy abnormal cells, so as to normalize the aforementioned abnormity in a body.

The molecular weight of a molecule of interest is, for example, 100 to 100,000, preferably 100 to 50,000, more preferably 100 to 25,000, further preferably 100 to 10,000, and most preferably 100 to 1,000.

The term "electron donor structure" is used in the present specification to mean a structure that easily releases electrons. The type of such an electron donor structure is not particularly limited, as long as it has a property of easily releasing electrons. An example of such an electron donor structure is a structure comprising a reducing agent such as a sulfur compound or a trivalent phosphorus compound. It is preferably a structure comprising diphenylphosphine, DTT (dithiothreitol), triphenylphosphine, alkylphosphine, etc. A method of obtaining an electron donor structure comprising a diphenylphosphine group or the like is not particularly limited. A commercially available product may be obtained, or a known synthesis method may be applied to produce such an electron donor structure.

The term "electron acceptor structure" is used in the present specification to mean a structure that easily accepts electrons. Moreover, the term "electron acceptor structure having a molecule of interest and an azide group" is used in the present specification to mean an electron acceptor structure, wherein a functional group having the aforementioned property, to which a molecule of interest has bound, is an azide group. If the azide group accepts electrons from the electron donor structure, a change in the structure occurs, and as a result, the structure releases the molecule of interest. The type of such an electron acceptor structure having a molecule of interest and an azide group is not particularly limited, as long as the molecule of interest has bound to the structure and the aforementioned property of easily accepting electrons can be achieved by the azide group. As such an electron acceptor structure, a structure represented by the following formula (8) is preferable, for example: 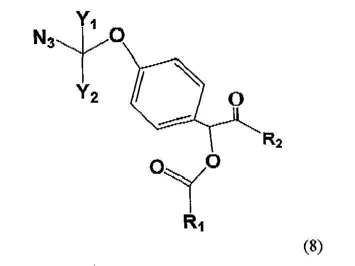 (wherein, in the above formula (8), each of Y₁ and Y₂ independently represents a hydrogen atom, an alkyl group containing 1 to 6 carbon atoms, an alkoxy group containing 1 to 6 carbon atoms, an aryl group containing 6 to 10 carbon atoms, or a cyano group; R₁ represents a residue of the molecule of interest; and R₂ represents a reactive group for binding to a nucleic acid.)

Examples of the "reactive group for binding to a nucleic acid" represented by R₂ in the present specification include a protected amide group, an amino group, a carboxylic acid group, an ethynyl group, halogen, an azide group, a thiol group, and an aldehyde group. These groups may have a linking group. Examples of a protecting group for protecting an amide group include urethane protecting groups such as a t-butoxycarbonyl group, acyl protecting groups such as a benzoyl group, alkyl protecting groups such as a trityl group, and imine protecting groups such as dimethylacetal. As a substituent, a reactive group capable of reacting with and binding to a nucleic acid is preferable. An example of such a reactive group is a halogen atom. Specific examples of a reactive group that binds to the nucleic acid of R₂ include a reactive group represented by formula (9) as shown below, -C≡C-CH₂-NHCO-CH₂Br, -N₃, -C≡CH, -SH, -NH₂, -CO₂H, -CHO, and the following groups.

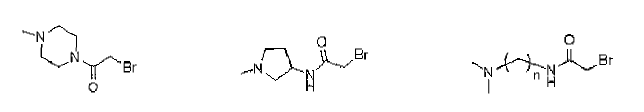

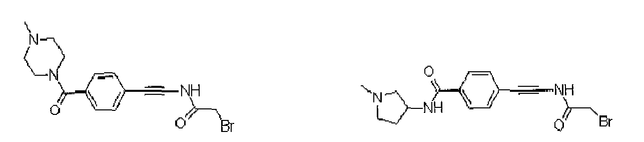

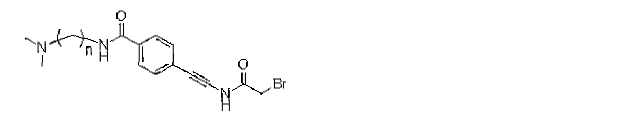

Bromoacetyl (BrCH2CO-) is important for DNA binding. As a linker between an electron acceptor and bromoacetyl, any given linkers can be used, as well as the aforementioned linkers. As such a linker between a bromoacetyl group and an electron acceptor, a saturated or unsaturated acyclic hydrocarbon containing 1 to 20 carbon atoms at a main chain thereof, an aliphatic or aromatic cyclic hydrocarbon containing 3 to 10 carbon atoms, or a complex thereof is preferable. Such a linker may have a substituent at a chain or ring thereof. Or, such a linker may also have a heteroatom (for example, an oxygen atom, a nitrogen atom, etc.) at a chain or ring thereof. Otherwise, two or more types of different linkers may be combined and used.

Examples of the aforementioned substituent include an alkyl group (a lower alkyl group containing 1 to 4 carbon atoms that may be branched), an acyl group, an aryl group, an alkoxyhydroxy group (a lower alkyl group containing 1 to 4 carbon atoms that may be branched), a keto group, an amino group (which may be substituted with a lower alkyl group containing 1 or 2 carbon atoms), an oxo group, an acetyl group, and a formyl group.

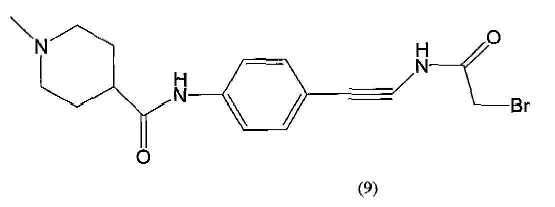

An example of the "alkyl group" used in the present specification is an alkyl group containing 1 to 6 carbon atoms, which may be linear or branched. Specific examples of such an alkyl group include a methyl group, an ethyl group, a propyl group, a butyl group, a pentyl group, and a hexyl group.

An example of the "acyl group" used in the present specification is an acyl group containing 1 to 6 carbon atoms. Specific examples of such an acyl group include an acetyl group and a propionyl group.

An example of the "aryl group" used in the present specification is an aryl group containing 6 to 10 carbon atoms. Specific examples of such an aryl group include a phenyl group and a naphthyl group.

An example of the "alkoxy group" used in the present specification is an alkoxy group containing 1 to 6 carbon atoms, which may be linear or branched. Specific examples of such an alkoxy group include a methoxy group, an ethoxy group, a propoxy group, a butoxy group, a pentyloxy group, and a hexyloxy group.

A method of obtaining an electron acceptor structure having a molecule of interest and an azide group is not particularly limited. For example, such an electron acceptor structure can be obtained by synthesizing according to the method described in the examples.

The term "electron donor-first nucleic acid probe molecule" is used in the present specification to mean a first nucleic acid probe having a nucleotide sequence complementary to a portion of a target nucleic acid sequence, to the 3'-terminal portion or 5'-terminal portion of which an electron donor structure has bound. Moreover, the term "3'-temrinal portion" is used in the present specification to mean a nucleotide sequence consisting of 1 to 10, preferably 1 to 5, and more preferably 1 to 3 nucleotides, which is located at the 3'-terminus, and the term "5'-temrinal portion" is used herein to mean a nucleotide sequence consisting of 1 to 10, preferably 1 to 5, and more preferably 1 to 3 nucleotides, which is located at the 5'-terminus, respectively.

The "electron donor-first nucleic acid probe" molecule may be obtained from the market place, or may be obtained by synthesis. When the electron donor-first nucleic acid probe molecules are obtained by synthesis, a nucleotide sequence complementary to a portion of a target nucleic acid sequence is determined, and a first nucleic acid probe is then synthesized based on a known oligonucleotide synthesis method such as a phosphoroamidite method. Thereafter, an electron donor structure that has been obtained by synthesis or from the market place, such as triphenylphosphine, is allowed to bind to the 3'-terminal portion or 5'-terminal portion of the nucleic acid probe. A method of binding the aforementioned electron donor structure to the terminus of the nucleic acid probe is not particularly limited. For example, the electron donor structure may be bound to the terminus by allowing it to react with a 5'-amino-modified oligo.

The term "molecule of interest-electron acceptor-second nucleic acid probe molecule" is used in the present specification to mean molecules obtained by binding a second nucleic acid probe that has a nucleotide sequence complementary to a nucleic acid sequence in the neighborhood separated at a certain distance from a portion of the aforementioned target nucleic acid sequence and differing from that of the aforementioned first nucleic acid probe to an electron acceptor structure having a molecule of interest and an azide group at the 5'-terminal portion or 3'-terminal portion thereof. The molecule of interest may be allowed to directly bind to the electron acceptor, or may be allowed to bind thereto via a linker. The binding site between the electron acceptor structure and the linker, or the binding site between the electron acceptor structure and the molecule of interest is preferably any one of O, N, and S. However, such a binding site is not particularly limited.

The type of the "linker" is not particularly limited, as long as it is capable of crosslinking the molecule of interest with the electron acceptor structure.

As such a linker, a saturated or unsaturated acyclic hydrocarbon containing 1 to 10 carbon atoms at a main chain thereof, which has a functional group acting as a binding site between the molecule of interest and the electron acceptor structure, an aliphatic or aromatic cyclic hydrocarbon containing 3 to 10 carbon atoms, or a complex thereof is preferable. Such a linker may have a substituent at a chain or ring thereof. Or, such a linker may also have a heteroatom (for example, an oxygen atom, a nitrogen atom, etc.) at a chain or ring thereof. Otherwise, two or more types of different linkers may be combined and used.

Examples of the aforementioned substituent include an alkyl group (a lower alkyl group containing 1 to 4 carbon atoms that may be branched), an acyl group, an aryl group, an alkoxyhydroxy group (a lower alkyl group containing 1 to 4 carbon atoms that may be branched), a keto group, an amino group (which may be substituted with a lower alkyl group containing 1 or 2 carbon atoms), an oxo group, an acetyl group, and a formyl group.

The molecule of interest-electron acceptor-second nucleic acid probe molecules may be obtained from the market place, or may be obtained by synthesis. When the electron acceptor probe is obtained by synthesis, a nucleotide sequence that is complementary to a desired sequence of a target nucleic acid and differs from that of the aforementioned first nucleic acid probe is determined, and a second nucleic acid probe is then synthesized based on a known oligonucleotide synthesis method such as a phosphoroamidite method. Thereafter, an electron donor structure having a molecule of interest and an azide group that has been obtained by synthesis or from the market place, for example, quencher-bound compound 19, is allowed to bind to the 3'-terminal portion or 5'-terminal portion of the nucleic acid probe. A method of binding the aforementioned electron acceptor structure having a molecule of interest and an azide group to the terminus of the nucleic acid probe is not particularly limited. For example, the electron acceptor structure may be bound to the terminus by allowing it to react with a 3'-phosphorothioate oligo. The details will be described in the example section.

The length of each of the aforementioned first and second nucleic acid probes is, for example, 5 to 1,000, preferably 5 to 100, more preferably 5 to 50, further preferably 5 to 25, and most preferably 8 to 15 nucleotides.

The binding site between the first nucleic acid probe and the electron donor structure in the electron donor-first nucleic acid probe molecules, and the binding site between the second nucleic acid probe and the electron acceptor structure having a molecule of interest and an azide group in the molecule of interest-electron acceptor-second nucleic acid probe molecules, are determined depending on a position at which each of the electron donor-first nucleic acid probe molecules and the molecule of interest-electron acceptor-second nucleic acid probe molecules hybridizes to the target nucleic acid sequence.

That is to say, when a nucleotide sequence complementary to the nucleotide sequence of the second nucleic acid probe is located closer to the 3'-terminal side of the target nucleic acid sequence than a nucleotide sequence complementary to the nucleotide sequence of the first nucleic acid probe is, the electron donor structure binds to the 5'-terminus of the first nucleic acid probe in the electron donor-first nucleic acid probe molecules, and the electron acceptor structure binds to the 3'-terminus of the second nucleic acid probe in the molecule of interest-electron acceptor-second nucleic acid probe molecules.

On the other hand, when a nucleotide sequence complementary to the nucleotide sequence of the second nucleic acid probe is located closer to the 5'-terminal side of the target nucleic acid sequence than a nucleotide sequence complementary to the nucleotide sequence of the first nucleic acid probe is, the electron donor structure binds to the 3'-terminal portion of the first nucleic acid probe in the electron donor-first nucleic acid probe molecules, and the electron acceptor structure binds to the 5'-terminal portion of the second nucleic acid probe in the molecule of interest-electron acceptor-second nucleic acid probe molecules.

Target nucleic acid sequence regions recognized by each of the electron donor-first nucleic acid probe molecules and the molecule of interest-electron acceptor-second nucleic acid probe molecules can be optionally determined, as long as it satisfies a condition that the azide group of the electron acceptor structure in the molecule of interest-electron acceptor-second nucleic acid probe molecules is reduced by the action of the electron donor structure in the electron donor-first nucleic acid probe molecules, when the two above probes hybridize to the target nucleic acid sequence. In order to satisfy the aforementioned condition, the target nucleic acid sequence regions recognized by each of the electron donor-first nucleic acid probe molecules and the molecule of interest-electron acceptor-second nucleic acid probe molecules are generally preferably adjacent to or close to each other. In order to satisfy the aforementioned condition, the target nucleic acid sequence regions recognized by each of the electron donor-first nucleic acid probe molecules and the molecule of interest-electron acceptor-second nucleic acid probe molecules are preferably to close to each other at a space of, for example, 1 to 20, preferably 1 to 10, more preferably 1 to 5, and further preferably 1 to 3 nucleotides.

The expression "the electron donor-first nucleic acid probe molecules act on the molecule of interest-electron acceptor-second nucleic acid probe molecules, so as to release the molecule of interest" is used in the present specification to mean that the electron donor structure of the electron donor-first nucleic acid probe molecules acts as a reducing agent and transfers electrons to the azide group of the electron acceptor structure of the adjacent molecule of interest-electron acceptor-second nucleic acid probe molecules, so as to cause a structure change to the electron acceptor structure, and as a result, the electron acceptor structure releases the molecule of interest.

The term "target nucleic acid sequence" is used in the present specification to mean the nucleotide sequence of a nucleic acid molecule acting as a target that determines a site at which the molecule of interest is to be released. For example, it is RNA or DNA, and preferably RNA. When the target nucleic acid sequence is RNA, it is preferably a linear sequence that does not have a secondary structure.

The method of the present invention for detecting a target nucleic acid sequence is a method for detecting a target nucleic acid sequence, which comprises: a step of hybridizing each of electron donor-first nucleic acid probe molecules formed by binding an electron donor structure to a first nucleic acid probe having a nucleotide sequence complementary to a target nucleic acid sequence and quencher-electron acceptor-fluorescent agent probe formed by binding an electron acceptor structure having a quencher and an azide group to a second nucleic acid probe having a nucleotide sequence complementary to the target nucleic acid sequence and differing from that of the first nucleic acid probe and a fluorescent agent, to the target nucleic acid sequence, and then allowing the electron donor-first nucleic acid probe molecules to act on the quencher-electron acceptor-fluorescent agent probe, so as to release the quencher; and a step of measuring the fluorescence of a complex obtained by the aforementioned hybridization. A specific example of the method of the present invention for detecting a target nucleic acid sequence is as shown in Figure 3.

The term "second nucleic acid probe having a fluorescent agent" is used in the present specification to mean the second nucleic acid probe having the aforementioned nucleotide sequence, to which a fluorescent agent has bound. A method of obtaining the second nucleic acid probe having a fluorescent agent is not particularly limited. It may be obtained from the market place, or may be obtained by performing synthesis according to a known method. Further, a site in the second nucleic acid probe, to which a fluorescent agent binds, is not particularly limited, as long as the quenching action of a quencher is available therein.

The term "electron acceptor structure having a quencher and an azide group" is used in the present specification to mean a structure in which the molecule of interest of said electron acceptor structure having a molecule of interest and an azide group is a quencher. A specific example of such an electron acceptor structure having a quencher and an azide group is represented by the following formula (10): 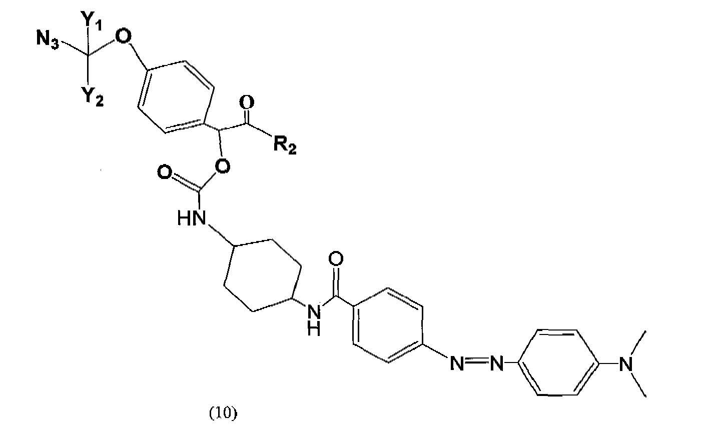 (wherein, in the above formula (10), each of Y₁ and Y₂ independently represents a hydrogen atom, an alkyl group containing 1 to 6 carbon atoms, an alkoxy group containing 1 to 6 carbon atoms, an aryl group containing 6 to 10 carbon atoms, or a cyano group; and R₂ represents a reactive group for binding to a nucleic acid.)

Moreover, the term "quencher-electron acceptor-fluorescent agent probe" is used in the present specification to mean a nucleic acid probe wherein a quencher is used as a molecule of interest and a fluorescent agent binds to a second nucleic acid probe in the aforementioned molecule of interest-electron acceptor-second nucleic acid probe molecules.

The types of a quencher and a fluorescent agent are not particularly limited in the quencher-electron acceptor-fluorescent agent probe. However, a combination, in which the quencher acts on the fluorescence developed by the fluorescent agent, is necessary. Such a combination is preferably dabcyl as a quencher and fluorescein as a fluorescent agent, for example.

The term "step of measuring the fluorescence of a hybridized complex" is used in the present specification to mean a step of measuring the fluorescence of a fluorescent agent that has bound to the second nucleic acid probe as a result of the release of the quencher. A method of measuring fluorescence is not particularly limited. For example, such fluorescence can be measured using a fluorospectrophotometer. In this case, an excitation wavelength is set at 490 nm, a fluorescence wavelength is set at 450 nm, and fluorescence contained in a sample can be then measured.

The compound of the present invention is represented by the following formula (11): 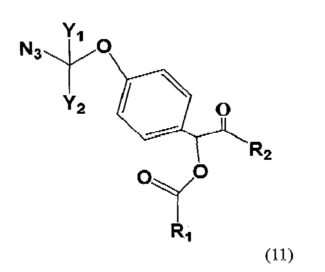 (wherein, in the above formula (11), each of Y₁ and Y₂ independently represents a hydrogen atom, an alkyl group containing 1 to 6 carbon atoms, an alkoxy group containing 1 to 6 carbon atoms, an aryl group containing 6 to 10 carbon atoms, or a cyano group; R₁ represents a residue of the molecule of interest; and R₂ represents a hydrogen atom, a halogen atom, or a reactive group for binding to a nucleic acid.)

Examples of the "halogen atom" used in the present specification include a fluorine atom, a chlorine atom, a bromine atom, and an iodine atom.

An example of the molecule of interest is a quencher, and is more specifically a compound represented by the following formula (12):

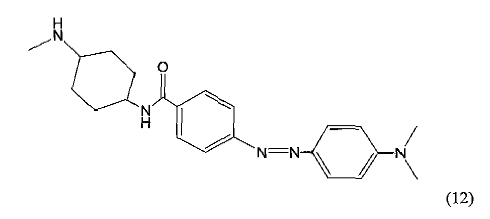

In a preferred embodiment, the compound of the present invention may be a compound represented by the following formula (13). A method of synthesizing the compound represented by the following formula (13) that is compound 19 is as shown in Figure 2.

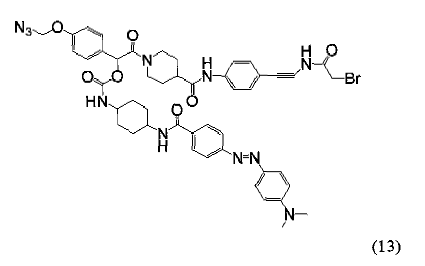

The compound of the present invention can be used in the method of the present invention for releasing a molecule of interest or in the method of the present invention for detecting a target nucleic acid sequence.

The present invention will be more specifically described in the following examples. However, these examples are not intended to limit the scope of the present invention.

### EXAMPLES

### [Example 1] Organic synthesis of compound of the present invention (compound 19 shown in Figure 2).

### (1) Synthesis of compound 3 (compound 3 shown in Figure 2)

Compound 1 (compound 1 shown in Figure 2) was protected with Boc according to the method described in the publication of Alexopoulos et al. (K. Alexopoulos et al., (2001), J. Med. Chem., 44, 328-338), so as to obtain compound 2 (compound 2 shown in Figure 2). Thereafter, compound 2 (2.2790 g; 9.9 mmol) and 4-iodoaniline (2.4156 g; 11.0 mmol; 1.1 eq) were dissolved in DMF (50 ml), and WSC (2.3130 g; 12.1 mmol; 1.2 eq) was then added to the solution. The mixture was stirred overnight. After the disappearance of compound 2 had been confirmed, the reaction solution was diluted with EtOAc. The resultant solution was separated with 2 N HCl (twice), and it was then washed with an NaCl saturated aqueous solution. The organic layer was dried over Na₂SO₄, and the residue was then purified with a silica gel column, so as to obtain compound 3 (4.1708 g; 9.7 mmol; 98%). 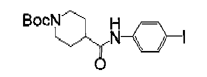 ¹H-NMR (400MHz, CDCl₃): δ 7.62-7.60, 7.31-7.29 (each 2H, d, *J=* 8.8, 8.6 Hz), 7.35 (1H, s), 4.17 (2H, m), 2.77 (2H, m), 2.37 (1H, m), 1.89 (2H, m), 1.75 (2H, m), 1.46 (9H, s).
¹³C-NMR (99.5MHz, CDCl₃): δ 172.46, 154.49, 137.79, 137.42, 121.58, 87.47, 79.81, 44.39,28.62,28.51.
QSTAR (Applied Biosystems/MDS SCIEX) (ESI): [MNa⁺] C₁₇H₂₃IN₂NaO₃: 453.0651, found: 453.0654.

### (2) Synthesis of compound 4 (compound 4 shown in Figure 2)

Compound 3 (2.9238 g; 6.8 mmol) was dissolved in CH₂Cl₂ (10 ml). TFA (30 ml) was added dropwise to the solution in an ice bath, and the reaction solution was then returned to room temperature, followed by stirring for 2 hours. After the disappearance of raw material had been confirmed, toluene was added to the reaction solution, and the solvent was then distilled away. EtOAc/Hexane was used to crystallize compound 4 (2.5692 g; 5.8 mmol; 85%). 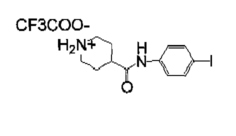 ¹H-NMR (400MHz, DMSO-d₆): δ 10.17 (1H, s), 8.75, 8.46 (each 1H, br), 3.35 (2H, m), 2.95-2.89 (2H, t, *J=* 12.0 Hz), 2.63 (1H, m), 1.97-1.93 (2H, d, *J=* 12.7 Hz), 1.80 (2H, m).
¹³C-NMR (99.5MHz, DMSO-d₆): δ 171.94, 138.67, 137.11, 121.24, 86.56, 42.41, 25.05. QSTAR (Applied Biosystems/MDS SCIEX) (ESI): [MH⁺] C₁₂H₁₆IN₂O: 331.0307, found: 331.0308.

### (3) Synthesis of compound 7 (compound 7 shown in Figure 2)

Compound 5 (compound 5 shown in Figure 2) was protected with Boc according to the method described in the publication of Komatsu et al. (T. Komatsu et al., (2006), J. Am. Chem. Soc., 128, 15946-15947), so as to obtain compound 6 (compound 6 shown in Figure 2). Compound 6 (0.166 g; 0.78 mmol; 1.2 eq) was dissolved in CH₂Cl₂ (30 ml). Thereafter, *p*-Methyl Red (0.177 g; 0.66 mmol), WSC (0.253 g; 1.32 mmol; 2 eq), and TEA (2 drops) were added to the solution, and the obtained mixture was then stirred overnight in an Ar atmosphere. After the disappearance of compound 6 had been confirmed using TLC (CHCl₃ : MeOH = 20 : 1), the residue was dissolved in CH₂Cl₂, and it was then separated with 2 N HCl and H₂O once each. The organic layer was dried over anhydrous NaSO₄, and the residue was then purified with a silica gel column, so as to obtain compound 7 (80.9 mg; 0.17 mmol; 26%). 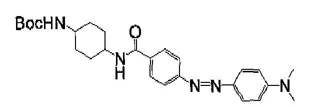 ¹H-NMR (400MHz, CDCl₃): δ 7.91-7.83 (6H, m), 6.77 (2H, d, *J*= 9.0 Hz), 6.12-5.98 (1H, m), 4.60-4.44 (1H, m), 4.12-3.98, 3.69-3.48 (each 1H, m), 3.11 (6H, s), 2.13-1.25 (8H, m), 1.46 (9H, s).
¹³C-NMR (99.5 MHz, CDCl₃): δ166.13, 154.87, 152.65, 143.49, 134.66, 127.60, 125.29, 122,16, 111.37, 48.29, 46.56, 40.35, 32.16, 31.94, 28.94, 28.52.
QSTAR (Applied Biosystems/MDS SCIEX) (ESI): [MH⁺] C₂₆H₃₆N₅O₄: 466.2818, found: 466.2805.

### (4) Synthesis of compound 8

Compound 7 (80 mg; 0.17 mmol) was dissolved in TFA (6 ml), and the mixture was then stirred. Two hours later, the disappearance of raw material was confirmed using TLC (CHCl₃ : MeOH = 20 : 1). Thereafter, toluene was added to the reaction solution, and the solvent was then distilled away, so as to obtain compound 8 (132.6 mg; 0.35 mmol, quant). 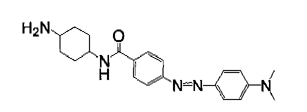 ¹H-NMR (400MHz, CD₃OD): δ 7.93-7.83 (6H, m), 6.84-6.82 (2H, d, *J*= 9.0 Hz), 3.90, 3.11 (each 1H, m), 2.11, 1.54 (each 4H, m).
¹³C-NMR (99.5 MHz, CD₃OD): δ167.08, 153.07, 142.69, 133.98, 127.85, 125.96, 121.34, 111.91, 77.44, 77.19, 47.53, 40.31, 29.98, 29.28.
QSTAR (Applied Biosystems/MDS SCIEX) (ESI): [MH⁺] C₂₁H₂₈N₅O₂: 366.2294, found: 366.2289.

### (5) Synthesis of compound 11 (compound 11 shown in Figure 2)

Compound 9 (compound 9 shown in Figure 2) was acetylated by the method described in Komatsu et al. (T. Komatsu et al., (2006), J. Am. Chem. Soc., 128, 15946-15947), so as to obtain compound 10 (compound 10 shown in Figure 2). Compound 10 (1.0039 g; 4.0 mmol) was dissolved in THF (16 ml). Thereafter, N-hydroxysuccinimide (0.7200 g; 6.3 mmol; 1.6 eq) and DCC (1.0314 g; 5.0 mmol; 1.3 eq) were then added to the solution, and the obtained mixture was then stirred overnight. After the disappearance of raw material had been confirmed, the reaction solution was filtrated to eliminate dicyclohexylurea. The solvent was distilled away, and the residue was then purified with a silica gel column, so as to obtain compound 11 (1.2282 g; 3.5 mmol; 88%). 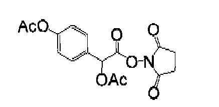 ¹H-NMR (400MHz, CDCl₃): δ 7.57-7.55, 7.19-7.16 (each 2H, d, *J*= 8.8, 8.8 Hz), 6.34 (1H, s), 2.81 (4H, s), 2.30, 2.19 (each 3H, s).
¹³C-NMR (99.5MHz, CDCl₃): δ 169.37, 168.83, 167.36, 151.66, 129.50, 129.37, 122.16, 71.73, 33.88, 25.62, 24.97, 21.22, 20.56.
QSTAR (Applied Biosystems/MDS SCIEX) (ESI): [MK⁺] C₁₆H₁₅KNO₈: 388.0435, found: 388.0418.

### (6) Synthesis of compound 12 (compound 12 shown in Figure 2)

Compound 4 (0.7909 g; 1.78 mmol) and compound 11 (0.8083 g; 2.31 mmol; 1.3 eq) were dissolved in THF (18 ml), and TEA (250 µl) was then added to the solution, followed by stirring. Three hours later, the disappearance of compound 11 was confirmed, and the reaction solution was then diluted with EtOAc. The resultant solution was separated with 2 N HCl twice, and it was then washed with an NaCl saturated aqueous solution. The organic layer was dried over Na₂SO₄. The solvent was distilled away, and the residue was then purified with a silica gel column, so as to obtain compound 12 (0.9090 g; 1.61 mmol; 90%). 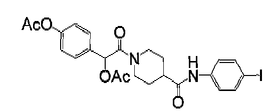 ¹H-NMR (400MHz, DMSO-d₆): δ 10.02-9.95 (1H, d, *J*= 28.3 Hz), 7.63-7.53 (4H, m), 7.45-7.43, 7.38-7.36 (each 1H, d, *J*= 8.56, 8.56 Hz) 7.21-7.16 (2H, m), 6.42-6.36 (1H, d, *J*= 21.5 Hz), 4.38-4.35, 4.11-3.94, 3.10, 2.85, 2.71-2.61, 1.56-1.48, 1.26-0.711 (each 1H, m), 2.27, 2.08 (each 3H, s), 1.81-1.73 (2H, m).
¹³C-NMR (99.5MHz, DMSO-d₆): δ 172.61, 169.47, 168.86, 165.66, 165.22, 150.62, 137.07, 131.80, 129.29, 121.99, 121.12, 86.30, 72.09, 71-67, 44.16, 42.57, 41.11, 28.11, 27.81, 20.85, 20.54,
QSTAR (Applied Biosystems/MDS SCIEX) (ESI): [MK⁺] C₂₄H₂₅IKN₂O₆: 603.0394, found: 603.0382.

### (7) Synthesis of compound 13 (compound 13 shown in Figure 2)

Compound 12 (0.5939 g; 1.05 mmol) was dissolved in 1,4-dioxane (15 ml), and 2 N NaOH (25 ml) was then added to the solution in an ice bath, followed by stirring. Five minutes later, the disappearance of raw material was confirmed, and HCl was then added dropwise to the reaction solution to adjust the pH value to pH 2.0. After the reaction solution had been extracted with EtOAc twice, it was washed with an NaCl saturated aqueous solution. The organic layer was dried over Na₂SO₄. The solvent was distilled away, and the residue was then purified with a silica gel column, so as to obtain compound 13 (0.2795 g; 0.58 mmol; 55%). 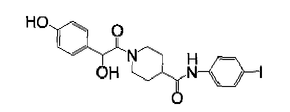 ¹H-NMR (400MHz, CD₃OD): δ7.59-7.57, 7.35-7.28, 7.22-7.18, 6.80-6.76 (each 2H, m), 5.36-5.30 (1H, d, *J*= 23.7 Hz) 4.62-4.59, 3.92-3.89, 2.99, 2.79-2.76, 2.51, 1.84-1.82, 1.66-1.64, 1.53-1.49, 0.94-0.92 (each 1H, m).
¹³C-NMR(99.5 MHz, CD₃OD): δ174.98, 158.65, 139.55, 138.67, 131.41, 129.67, 122.90, 116.59, 87.62, 72.63, 61.50, 45.56, 44.56, 43.14, 20.91, 14.53.
QSTAR (Applied Biosystems/MDS SCIEX) (ESI): [MNa⁺] C₂₀H₂₁IN₂NaO₄: 503.0444, found: 503.0436.

### (8) Synthesis of compound 14 (compound 14 shown in Figure 2)

Compound 13 (0.2795 g; 0.58 mmol) was dissolved in DMF (1.3 ml), and NaI (15.9 mg; 0.11 mmol; 0.18 eq) was then added to the solution, followed by stirring. After the reaction solution had been cooled on ice, KO*t*-Bu (97.2 mg; 0.86 mmol; 1.5 eq) dissolved in THF (1.5 ml) was added dropwise thereto. Thereafter, CH₃SCH₂Cl (73 µl; 0.87 mmol; 1.5 eq) was added to the solution, and the obtained mixture was then stirred at room temperature. 4.5 hours later, the disappearance of raw material was confirmed using TLC. The reaction solution was diluted with EtOAc, and it was then separated with H₂O (twice). Thereafter, it was washed with an NaCl saturated aqueous solution. The organic layer was dried over anhydrous NaSO₄, and the solvent was then distilled away. The residue was purified with a silica gel column, so as to obtain compound 14 (0.2467 g; 0.46 mmol; 78%). 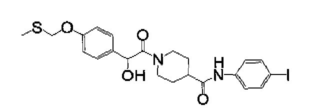 ¹H-NMR (400MHz, DMSO-d₆): δ 9.99-9.96 (1H, d, *J*= 13.9 Hz), 7.60, 7.43-7.35, 7.29-7.23, 6.98 (each 2H, m), 5.43-5.27 (1H, m), 5.43-5.27 (1H, m), 5.25 (2H, s), 2.15 (3H, s), 4.43-4.40, 3.97-3.92, 2.92, 2.76-2.65, 1.79, 1.63, 1.52-1.46, 1.34, 0.93 (each 1H, m).
¹³C-NMR (99.5MHz, DMSO-d₆): δ 172.65, 170.04, 163.56, 138.20, 137.05, 133.25, 127.70, 121.11, 115.64, 86.31, 70.87, 59.69, 43.75, 43.46, 41.29, 27.64, 20.78, 14.12. QSTAR (Applied Biosystems/MDS SCIEX) (ESI): [MNa⁺] C₂₂H₂₅IN₂NaO₄S: 563.04777, found: 563.0469.

### (9) Synthesis of compound 15 (compound 15 shown in Figure 2)

Compound 14 (0.2685 g; 0.50 mmol) was dissolved in CH₂Cl₂ (10 ml), and NCS (85.5 mg; 0.64 mmol; 1.3 eq) was then added to the solution, followed by stirring. Five minutes later, TMSCl (76 µl; 0.59 mmol; 1.2 eq) was added to the reaction solution. One hour later, the reaction solution was diluted with CHCl₃, and it was then separated with saturated NaHCO₃ (twice). Thereafter, it was washed with an NaCl saturated aqueous solution. The organic layer was dried over anhydrous NaSO₄, and the solvent was then distilled away. The residue was dissolved in DMF (9 ml), and NaN₃ (49.8 mg; 0.77 mmol; 1.5 eq) dissolved in H₂O (5 ml) was then added to the solution. The obtained mixture was then stirred. 1.5 hours later, saturated NaHCO₃ was added to the reaction solution. The mixed solution was extracted with EtOAc twice, and it was then washed with an NaCl saturated aqueous solution. The organic layer was dried over Na₂SO₄. The solvent was distilled away, and the residue was then purified with a silica gel column, so as to obtain compound 15 (85.6 mg; 0.16 mmol; 32%). 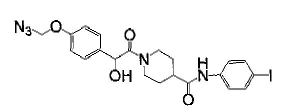 ¹H-NMR (400MHz, DMSO-d₆): δ 9.98-9.95 (1H, d, *J*= 13.9 Hz), 7.60, 7.43-7.38, 7.33-7.31, 7.04-7.02 (each 2H, m), 5.49-5.47 (1H, m), 5.38 (2H, s), 4.43-4.39, 4.06-3.93, 3.65, 2.92-2.89, 2.78-2.66, 1.63, 1.53-1.46, 1.35-1.32 (each 1H, m).
¹³C-NMR (99.5MHz, DMSO-d₆): δ 172.64, 170.12, 155.24, 138.81, 137.04, 134.30, 128.80, 127.94, 121.13, 115.54, 86.30, 78.78, 70.58, 70.18, 59.69, 43.82, 42.44, 41.29, 33.27, 28.42, 28.06, 27.92, 22.63, 20.78, 15.00, 14.78, 14.11.
QSTAR (Applied Biosystems/MDS SCIEX) (ESI): [MK⁺] C₂₁H₂₂IKN₅O₄: 574.0354 , found: 574.0335.

### (10) Synthesis of compound 17

Compound 15 (84.9 mg; 0.16 mmol) was dissolved in CH₂Cl₂ (8 ml), and 4-nitrophenyl chloroformate (129 mg; 0.64 mmol; 4 eq) and TEA (220 µl; 1.58 mmol; 9.9 eq) were then added to the solution. Three hours later, the disappearance of raw material was confirmed using TLC. The reaction solution was diluted with CHCl₃, and it was then separated with H₂O (twice). Thereafter, it was washed with an NaCl saturated aqueous solution. The organic layer was dried over anhydrous NaSO₄, and the solvent was then distilled away. The residue was purified with a silica gel column, so as to obtain compound 16 (compound 16 shown in Figure 2).

Compound 16 was dissolved in CH₂Cl₂ (8 ml), and compound 8 (44.2 mg; 0.092 mmol; 0.5 eq) and TEA (100 µl; 0.717 mmol; 4.5 eq) were then added to the solution, followed by stirring overnight. Thereafter, the reaction solution was heated at 35°C for 2.5 hours. Subsequently, the disappearance of compound 16 was confirmed using TLC. The reaction solution was diluted with CHCl₃, and it was then separated with H₂O (twice). Thereafter, it was washed with an NaCl saturated aqueous solution. The organic layer was dried over anhydrous NaSO₄, and the solvent was then distilled away. The residue was purified with a column, so as to obtain compound 17 (52.8 mg; 0.057 mmol; 36% (2 steps)). 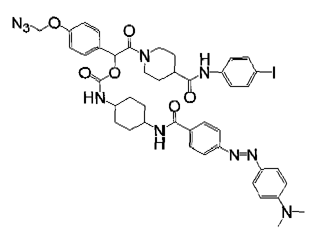 ¹H-NMR (400MHz, CDCl₃): δ7.89-7.85 (6H, t, *J*= 8.2 Hz), 7.59-7.57, 7.40-7.29, 7.04-7.02, 6.79-6.77 (each 2H, m), 6.25-6.22 (1H, d), 6.11 (1H, m), 5.19 (2H, s), 4.55 (1H, m), 3.94 (2H, m), 3.45-3.37 (4H, m), 3.12 (6H, s), 2.85-2.78 (2H, m), 2.47 (1H, m), 2.09 (4H, m), 1.91-1.62 (4H, m), 1.40 (6H, m).
¹³C-NMR (99.5MHz, CDCl₃): δ 172.76, 167.50, 166.93, 154.62, 152.62, 143.18, 137.84, 137.41, 134.38, 129.42, 127.67, 125.11, 121.76, 121.40, 116.13, 111.25, 86.97, 79.32, 72.17, 44.40, 42.83, 42.09, 40.09, 31.21, 29.58, 28.01.
QSTAR (Applied Biosystems/MDS SCIEX) (ESI): [MH⁺] C₄₃H₄₈IN₁₀O₆: 927.2803, found: 927.2801.

### (11) Synthesis of compound 19 (compound 19 shown in Figure 2)

Compound 17 (52.8 mg; 0.057 mmol) was dissolved in DMF (1.1 ml). Thereafter, compound 18 (compound 18 shown in Figure 2; 52.8 mg; 0.302 mmol; 5.3 eq) used as a bromoacetyl linker, tetrakis(triphenylphosphine) Pd (6.6 mg; 0.006 mmol; 0.1 eq), CuI (2.0 mg; 0.011 mmol; 0.2 eq), and TEA (40 µl; 0.287 mmol; 5.0 eq) were added to the solution. Thirty minutes later, the disappearance of raw material was confirmed using TLC. The solvent was distilled away, and the residue was then purified with a silica gel column, so as to obtain compound 19 (48.1 mg; 0.049 mmol; 87%). 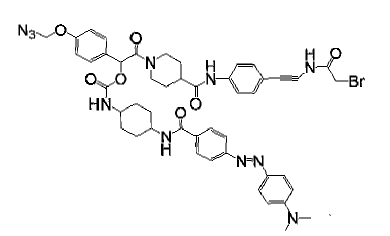 ¹H-NMR (400MHz, DMSO-d₆): δ10.09-10.02 (1H, d, *J*= 26.1 Hz), 8.80, 8.33 (each 1H, m), 7.83-7.79 (6H, t, *J*= 7.9 Hz), 7.63-7.61, 7.47-7.45, 7.33,7.07 (each 2H, m), 6.86-6.84 (2H, d, *J*= 9.0 Hz), 6.24-6.20 (1H, d, *J*= 15.4 Hz), 5.41 (2H, s), 4.37 (1H, m), 4.14-4.03 (4H, m), 3.89 (2H, s), 3.73-3.67 (1H, m), 3.32 (4H, br), 3.11 (6H, s), 2.89-2.68 (1H, m), 1.86-1.57 (6H, m), 1.42-1.23 (6H, m).
¹³C-NMR (99.5MHz, DMSO-d₆): δ 165.49, 164.58, 153.60, 152.56, 142.39, 134.74, 131.79, 129.64, 128.18, 124.85, 121.20, 118.76, 115.66, 111.40, 78.68, 31.36, 30.91, 29.26, 29.09, 25.51, 0.15.
QSTAR (Applied Biosystems/MDS SCIEX) (ESI): [MH⁺] C₄₈H₅₃BrN₁₁O₇: 974.3313, found: 974.3301.

### [Example 2] Synthesis of oligonucleotides

All oligonucleotides were synthesized according to a common phosphoroamidite method using 0.2 µM-scale column, employing a DNA automatic synthesizer (H-8-SE; Gene World). Deprotection of nucleotides and cleavage thereof from a CPG carrier were carried out by incubation in ammonia water at 55°C for 4 hours. Such oligonucleotide was purified using a reversed phase column (MicroPure II; Biosearch Technologies). The concentration was determined by measuring UV absorbance.

### [Example 3] Production of DNA probe to which compound of the present invention (compound 19 shown in Figure 2) has bound

Binding of compound 19 was carried out by reaction with 3'-phosphorothioate oligo. Such 3'-phosphorothioate oligo was synthesized by performing the coupling of 3'-phosphate CPG with an initial monomer and then converting the obtained product to 3'-phosphorothioate oligo using a sulfurizing reagent (Glen research). The reaction was carried out by intensively stirring a mixed solution comprising 3 mM compound 19 (in DMF), a 30-mM NaB buffer and a 300-µM 3'-phosphorothioate oligo solution at room temperature for 5 hours (DMF concentration in the reaction solution: 60%). Thereafter, the reaction solution was diluted with Milli Q, and it was then purified by reverse phase HPLC (gradient conditions: 0%-100% acetonitrile/50 mM triethylammonium acetate). Moreover, it was confirmed by MALDI-TOF mass spectrometry that a product of interest was obtained. 5'-AAG^{Flu}TGCTT ^{compound 19}-3'_{:} calculated mass, C155H177N42O63P8S 3915.2; found 3929.0.

### [Example 4] Reaction on DNA template and fluorescence measurement

A DNA probe, 5'-AAG^{Flu}TGCTT^{compound 19}-3', produced in Example 3 by binding the compound 19 having dabcyl and an azide group to a fluorescent agent, a DNA probe, 5'-^{TPP}TTG AAC TC-3', to the 5'-terminal side of which a triphenylphosphine group had bound, and a DNA template as shown in SEQ ID NO: 1 of the sequence listing were reacted (Figure 4A), and thereafter, fluorescence was measured. The reaction was carried out by reacting each 50 nM the DNA template, the 5'-triphenylphosphine-bound probe and the compound 19-bound probe at 37°C in a Ligation buffer (20 mM Tris-HCl, 100 mM MgC12, and 0.01 mg/ml BSA; pH 7.2). In order to confirm generation of a signal specific for the DNA template, a change in a fluorescent signal over time was measured even under a condition in which no DNA templates were present, and a comparison was then made (Figure 4B).

Such a fluorescent signal was analyzed using a fluorospectrophotometer (FP-6500; JASCO). Fluorescence was measured after 30, 90, and 180 minutes have passed. An excitation wavelength was set at 490 nm, and a fluorescence wavelength was set at 450 nm.

As a result, when the DNA template was present, a fluorescent signal was increased. In contrast, when the DNA template was absent, almost no increase in the fluorescent signal was observed (Figure 4B). Accordingly, it was revealed that the DNA probe set released a quencher as a molecule of interest, and that it generated a fluorescent signal specifically for a target nucleic acid sequence. That is, from the results of the present examples, it was revealed that the present invention enables the release of a molecule of interest that has bound to a second nucleic acid probe specifically for the target nucleic acid sequence.

### [Example 5]

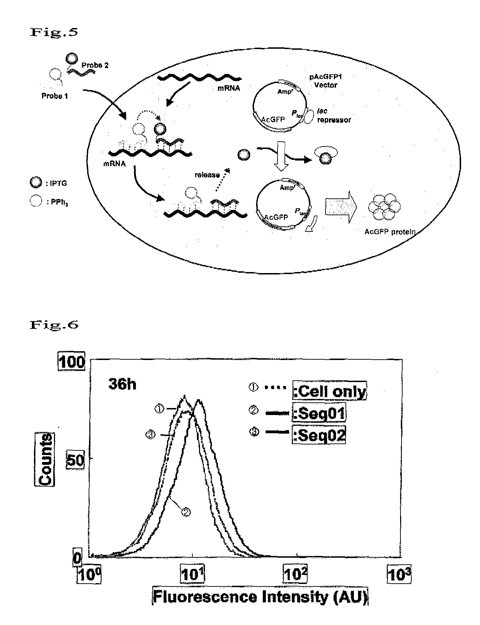

An attempt was made to release an agent based on intracellular genetic information. IPTG (Isopropyl β-D-1-thiogalactopyranoside) was used as a molecule to be released. By such release, expression of a protein was induced. In the present experiment, the effect of such molecule release was analyzed based on the expression level of an AcGFP fluorescent protein in *Escherichia coli* (Figure 5).

### [Experimental method]

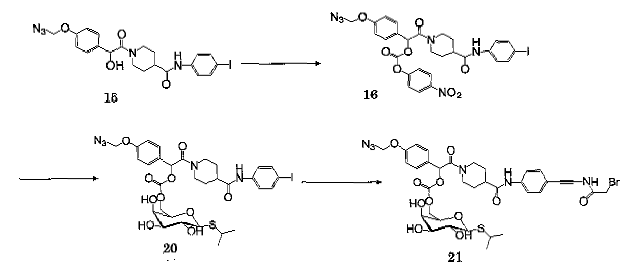

### (1) Synthesis of compound 20

Compound 15 (0.2060 g; 0.38 mmol) was dissolved in CH₂Cl₂ (20 ml), and 4-nitrophenyl chloroformate (0.3893 g; 1.93 mmol; 5.0 eq) and TEA (490 µl; 3.52 mmol; 9.1 eq) were then added to the solution. The reaction solution was stirred for 2.5 hours. Thereafter, the reaction solution was diluted with CHCl₃, and it was then separated from water, followed by washing with a saline solution. The organic layer was dried over Na₂SO₄, and the solvent was then distilled away. The residue was purified by flash chromatography to obtain compound 16. Compound 16 was dissolved in pyridine (4 ml), and IPTG (0.0953 g; 0.40 mmol; 1.0 eq) and DMAP (7.1 mg; 0.06 mmol; 0.2 eq) were then added to the solution. The reaction solution was stirred for 18 hours. Thereafter, the reaction solution was diluted with ethyl acetate, and it was then separated from water, followed by washing with a saline solution. The organic layer was dried over Na₂SO₄, and the solvent was then distilled away. The residue was purified by flash chromatography to obtain compound 20 (78,2 mg; 0.10 mmol; 25% (2steps)).
QSTAR (Applied Biosystems/MDS SCIEX) (ESI-Q-TOF): [MNa⁺] C₃₁H₃₆BrN₅NaO₁₀S : 822.1276 , found: 822.1299.

### (2) Synthesis of compound 21

Compound 20 (27.7 mg; 0.053 mmol) and 2-bromo-N-(propargyl)acetamide (31.6 mg; 0.18 mmol; 5.2 eq) were dissolved in DMF (0.7 ml). Thereafter, tetrakis(triphenylphosphine)palladium (5.4 mg; 0.005 mmol; 0.1 eq), copper (I) iodide (2.1 mg; 0.01 mmol; 0.3 eq), and TEA (24 µl; 0.17 mmol; 5.0 eq) were added to the solution. The reaction solution was stirred for 1 hour. Thereafter, the solution was concentrated, and the residue was then purified by flash chromatography to obtain compound 21 (19.3 mg; 0.02 mmol; 66%).
QSTAR (Applied Biosystems/MDS SCIEX) (ESI-Q-TOF): [MNa⁺]
C₃₆H₄₃BrN₆NaO₁₁S: 869.1786, found: 869.1767.

### (3) Production of DNA probe to which compound 21 has bound

23srRNA was used as a target nucleic acid sequence (Bernhard M. Fuchs et al, Applied and Environmental Microbiology, Feb. 2001, p.961-968). As a compound 21-bound DNA probe and an electron donor probe, two types of probes, namely, a match sequence (Seq01) and a mismatch sequence (Seq02), were synthesized (SEQ ID NOS: 2 to 4).

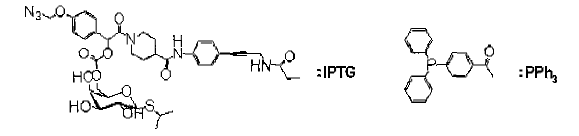

target
sequence : 28srRNA(position:968-998)
3'-CGACCGCCAGACCCA · · · · A · · · · CAAAGGGAGAAGTGC-5'
5'-GCTGGCGGTCTGGGT-IPTG-3'    5'-PPh3-GTTTCCCTCTTCACG-3' : Seq01
5'-PPh3-GCCTTCTCCCGAAGT-3' : Seq02

Binding of compound 21 was carried out by reaction with 3'-phosphorothioate oligo. Such 3'-phosphorothioate oligo was synthesized by performing the coupling of 3'-phosphate CPG with an initial monomer and then converting the obtained product to 3'-phosphorothioate oligo using a sulfurizing reagent. The reaction was carried out by intensively stirring a mixed solution comprising 3 mM compound 21 (in DMF), a 80-mM TEAA buffer and a 200-µM 3'-phosphorothioate oligo solution at room temperature for 5 hours (DMF concentration in the reaction solution: 80%). Thereafter, the reaction solution was diluted with Milli Q, and it was then purified by reverse phase HPLC (gradient conditions: 0%-100% acetonitrile/50 mM TEAA buffer). Moreover, it was confirmed by MALDI-TOF mass spectrometry that a product of interest was obtained.
5'-GCTGGCGGTCTGGGT-IPTG-3': calculated mass, C₁₈₃H₂₂₇N₆₃O₁₀₅P₁₅S₂ 5514.99; found 5519.36.

### (4) Introduction of probe into Escherichia coli

pAcGFP1 vector-transformed *Escherichia coli* JM109 was pre-cultured at 37°C in an LB/Amp medium overnight. A cell mass was recovered so that OD₆₀₀=0.6, and the recovered cell mass was then suspended in 50 µl of a buffer (0- or 50-µM probe, 20 mM Tris-HCl (pH7.2), 0.9 M NaCl, and 0.1% SDS). The suspension was incubated at 37°C for 30 minutes, and 950 µl of an SOC medium was then added to the reaction solution, followed by further incubation for 1 hour. One hour later, the cell mass was concentrated to 100 µl, and 900 µl of an LB/Amp medium was then added thereto. The obtained mixture was subjected to shaking and stirring at 37°C. Thirty-six hours later, the cell mass was recovered, followed by an FACS measurement.

### [Results]

The results of FACS measurement are shown in Figure 6. In the case of using Seq02 that is a random sequence, the expression level of a GFP protein was almost equivalent to that of the case where no IPTG had been added (cell only). This result demonstrated that IPTG was not released in the case of Seq02. In contrast, in the case of using Seq01 that is a match sequence, an increase in the fluorescence was clearly observed when compared with the cell only case. Thus, it was confirmed that IPTG was released sequence-specifically. These results demonstrated that the developed probe enables the release of an agent in cells, gene-sequence-specifically.

## Claims

1. A method for releasing a molecule of interest, which comprises steps of:
hybridizing each of an "electron donor-first nucleic acid probe" molecule formed by binding an electron donor structure to a first nucleic acid probe having a nucleotide sequence complementary to a portion of a target nucleic acid sequence and a "molecule of interest-electron acceptor-second nucleic acid probe" molecule formed by binding an electron acceptor structure having a molecule of interest and an azide group to a second nucleic acid probe that has a nucleotide sequence complementary to said target nucleic acid sequence and differing from that of said first nucleic acid probe, to said target nucleic acid sequence; and
allowing said "electron donor-first nucleic acid probe" molecule to act on said "molecule of interest-electron acceptor-second nucleic acid probe" molecule, so as to release said molecule of interest.

2. The method for releasing a molecule of interest according to claim 1, wherein, the nucleotide sequence complementary to the nucleotide sequence of said second nucleic acid probe is located closer to the 3'-texminal side of said target nucleic acid sequence than a nucleotide sequence complementary to the nucleotide sequence of said first nucleic acid probe is;
said electron donor structure binds to the 5'-terminal portion of said first nucleic acid probe in said electron donor-first nucleic acid probe molecule; and
said electron acceptor structure binds to the 3'-terminal portion of said second nucleic acid probe in said molecule of interest-electron acceptor-second nucleic acid probe molecule.

3. The method for releasing a molecule of interest according to claim 1 or 2, wherein,
the nucleotide sequence complementary to the nucleotide sequence of said second nucleic acid probe is located closer to the 5' -terminal side of said target nucleic acid sequence than a nucleotide sequence complementary to the nucleotide sequence of said first nucleic acid probe is;
said electron donor structure binds to the 3'-terminal portion of said first nucleic acid probe in said electron donor-first nucleic acid probe molecules; and said electron acceptor structure binds to the 5'-terminal portion of said second nucleic acid probe in said molecule of interest-electron acceptor-second nucleic acid probe molecules.

4. The method for releasing a molecule of interest according to any of claims 1 to 3, wherein the nucleotide sequence complementary to the nucleotide sequence of said second nucleic acid probe is located directly next to or 1 to 20 nucleotides away from a nucleotide sequence complementary to the nucleotide sequence of said first nucleic acid probe.

5. The method for releasing a molecule of interest according to any of claims 1 to 4, wherein said electron donor structure is a structure comprising a reducing agent.

6. The method for releasing a molecule of interest according to claim 5, wherein said reducing agent is a reducing agent comprising a diphenylphosphine group.

7. The method for releasing a molecule of interest according to any of claims 1 to 6, wherein said electron acceptor structure is represented by the following formula (1): 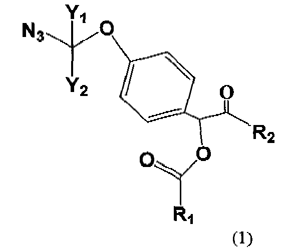 wherein, in the above formula (1), each of Y₁ and Y₂ independently represents a hydrogen atom, an alkyl group containing 1 to 6 carbon atoms, an alkoxy group containing 1 to 6 carbon atoms, an aryl group containing 6 to 10 carbon atoms, or a cyano group; R₁ represents a residue of the molecule of interest; and R₂ represents a reactive group for binding to a nucleic acid.

8. The method for releasing a molecule of interest according to claim 7 wherein the R₂ is a reactive group represented by the following formula (2).

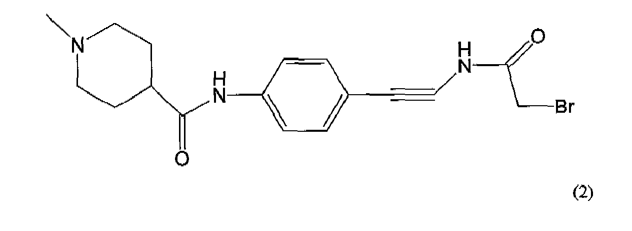

9. The method for releasing a molecule of interest according to any of claims 1 to 8, wherein the molecule of interest is a poison, an agent, or a quencher.

10. The method for releasing a molecule of interest according to claim 9, wherein the agent is IPTG (isopropyl β-D-1-thiogalactopyranoside), and the quencher is dabcyl.

11. A method for detecting a target nucleic acid, which comprises:
a step of hybridizing each of an "electron donor-first nucleic acid probe" molecule formed by binding an electron donor structure to a first nucleic acid probe having a nucleotide sequence complementary to a target nucleic acid sequence, and a "quencher-electron acceptor-fluorescent agent probe" formed by binding an electron acceptor structure having a quencher and an azide group to a second nucleic acid probe that has a nucleotide sequence complementary to said target nucleic acid sequence and differing from that of said first nucleic acid probe and a fluorescent agent, to said target nucleic acid sequence, and then allowing said "electron donor-first nucleic acid probe" molecule to act on said "quenchex-electron acceptor-fluorescent agent probe", so as to release said quencher; and
a step of measuring the fluorescence of a complex obtained by said hybridization.

12. The method for detecting a target nucleic acid according to claim 11, wherein, the nucleotide sequence complementary to the nucleotide sequence of said second nucleic acid probe is located closer to the 3'-terminal side of said target nucleic acid sequence than a nucleotide sequence complementary to the nucleotide sequence of said first nucleic acid probe is;
said electron donor structure binds to the 5'-terminal portion of said first nucleic acid probe in said electron donor-first nucleic acid probe molecules; and
said electron acceptor structure binds to the 3'-terminal portion of said second nucleic acid probe in said quencher-electron acceptor-fluorescent agent probe.

13. The method for detecting a target nucleic acid according to claim 11 or 12, wherein,
the nucleotide sequence complementary to the nucleotide sequence of said second nucleic acid probe is located closer to the 5'-terminal side of said target nucleic acid sequence than a nucleotide sequence complementary to the nucleotide sequence of said first nucleic acid probe is;
said electron donor structure binds to the 3'-terminal portion of said first nucleic acid probe in said electron donor-first nucleic acid probe molecules; and
said electron acceptor structure binds to the 5'-terminal portion of said second nucleic acid probe in said quencher-electron acceptor-fluorescent agent probe.

14. The method for detecting a target nucleic acid according to any of claims 11 to 13, wherein the nucleotide sequence complementary to the nucleotide sequence of said second nucleic acid probe is located directly next to or 1 to 20 nucleotides away from a nucleotide sequence complementary to the nucleotide sequence of said first nucleic acid probe.

15. The method for detecting a target nucleic acid according to any of claims 11 to 14, wherein said electron donor structure is a structure comprising a reducing agent.

16. The method for detecting a target nucleic acid according to claim 15, wherein said reducing agent is a reducing agent comprising a diphenylphosphine group.

17. The method for detecting a target nucleic acid according to any of claims 11 to 16, wherein said electron acceptor structure is a compound represented by the following formula (3): 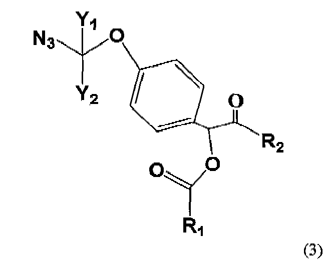 wherein, in the above formula (3), each of Y₁ and Y₂ independently represents a hydrogen atom, an alkyl group containing 1 to 6 carbon atoms, an alkoxy group containing 1 to 6 carbon atoms, an aryl group containing 6 to 10 carbon atoms, or a cyano group; R₁ represents a residue of the quencher; and R₂ represents a reactive group for binding to a nucleic acid.

18. The method for detecting a target nucleic acid according to claim 17, wherein the R₂ is a reactive group represented by the following formula (4):

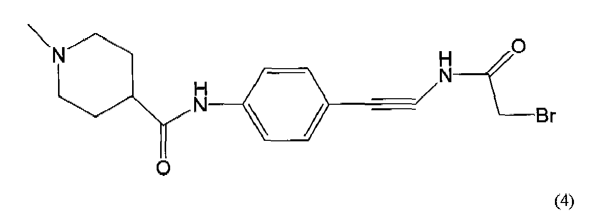

19. The method for detecting a target nucleic acid according to any of claims 11 to 18, wherein the quencher is dabcyl,

20. The method for detecting a target nucleic acid according to any of claims 11 to 19, wherein the fluorescent agent is fluorescein.

21. A compound represented by the following formula (5): 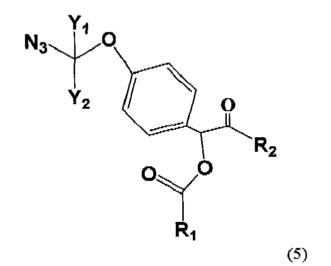 wherein, in the above formula (5), each of Y₁ and Y₂ independently represents a hydrogen atom, an alkyl group containing 1 to 6 carbon atoms, an alkoxy group containing 1 to 6 carbon atoms, an aryl group containing 6 to 10 carbon atoms, or a cyano group; R₁ represents a residue of the molecule of interest; and R₂ represents a hydrogen atom, a halogen atom, or a reactive group for binding to a nucleic acid.

22. The compound according to claim 21, wherein the R₁ is a quencher.

23. The compound according to claim 22, wherein the quencher is a quencher represented by the following formula (6):

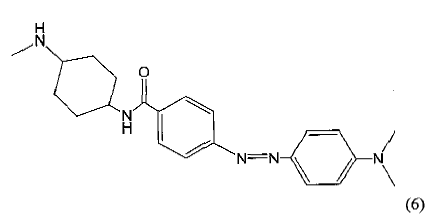

24. A compound represented by the following formula (7):

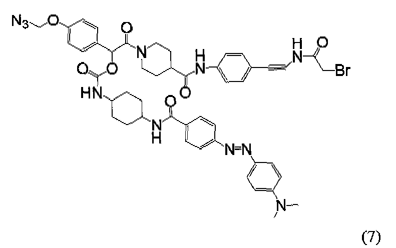

25. The compound of any of claims 21 to 24 for use in the method for releasing a molecule of interest according to claims 1 to 10 or in the method for detecting a target nucleic acid sequence according to any one of claims 11 to 20.
